# EUROPEAN PATENT APPLICATION

(11) **EP 3 848 043 A1**
(43) Date of publication of application: **14.07.2021**
(21) Application number: 19856844.6
(22) Date of filing: 04.09.2019
(51) Int. Cl.: A61K 36/704, A61K 36/47, A61P 29/00, A61P 37/08, A61P 11/06, A61P 11/00, A23L 33/105

(54) **COMPOSITION FOR IMPROVING RESPIRATORY DISEASE INCLUDING EXTRACT OF PALIURUS RAMOSISSIMUS (LOUR.) POIR**

(30) Priority: 04.09.2018 KR 20180105187; 04.09.2018 KR 20180105188; 04.09.2018 KR 20180105189; 04.09.2018 KR 20180105190; 04.09.2018 KR 20180105191; 04.09.2018 KR 20180105192
(71) Applicant: Korea Food Research Institute, Wanju-gun, Jeollabuk-do 55365 (KR)
(72) Inventor: SHIN, Hee Soon, Jeonju-si Jeollabuk-do 54871 (KR); KIM, Seung Yong, Jeonju-si Jeollabuk-do 55079 (KR); SHIN, Dong Uk, Cheongju-si Chungcheongbuk-do 28380 (KR); EOM, Ji Eun, Jeonju-si Jeollabuk-do 54964 (KR); LEE, So Young, Jeonju-si Jeollabuk-do 54865 (KR); JUNG, Sun Young, Jeonju-si Jeollabuk-do 54961 (KR); CHOI, Dae Woon, Taebaek-si Gangwon-do 26043 (KR); NAM, Young Do, Jeonju-si Jeollabuk-do 55063 (KR); PARK, So Lim, Jeonju-si Jeollabuk-do 54867 (KR)
(74) Representative: Algemeen Octrooi- en Merkenbureau B.V.
(86) International application number: PCT/KR2019/011432
(87) International publication number: WO 2020/050633

(57) **Abstract**

The present invention discloses a composition for alleviating lung diseases, such as asthma and chronic obstructive pulmonary disease, using the extracts of Paliurus ramosissimus (Lour.) Poir, Rosa davurica Pall., Picrasma quassioides, Camellia sinensis, Chrysosplenium grayanum, Reynoutria japonica, Pseudocydonia sinensis, Hydrangea serrata, and/or Euonymus alatus. The extracts of Paliurus ramosissimus (Lour.) Poir, Rosa davurica Pall., Picrasma quassioides, Camellia sinensis, Chrysosplenium grayanum, Reynoutria japonica, Pseudocydonia sinensis, Hydrangea serrata, and/or Euonymus alatus have the activities of inhibiting the Netosis activation of neutrophil extracellular traps induced by phorbol myristate acetate (PMA), inhibiting the expression of IL-8, an inflammatory cytokine by CSE of NCI-H292 cells, a bronchial epithelial cell line producing mucus, and inhibiting the expression of MIP-2, an inflammatory cytokine of alveolar macrophages MH-S cells activated by CES and LPS (lipopolysaccaride). In addition, the extracts have the activities of inhibiting lung tissue damage in the mouse model of CSE and PPE induced respiratory disease, inhibiting immune cell infiltration of total cell, macrophages, lymphocytes, eosinophils, and neutrophils in BALF, and inhibiting the secretion of inflammatory cytokines and chemokines in BALF and lung tissue.

## Description

### [Technical Field]

The present disclosure relates to a composition for alleviating allergy, asthma or chronic obstructive pulmonary disease (COPD), using extract of Paliurus ramosissimus (Lour) Poir and the like.

### [Background Art]

The allergy reaction is classified into five types of type I anaphylaxis, type II cytolysis, type III immune complex, type IV cell mediation, and type V stimulatory hypersensitivity reaction, and most of which belong to type I, and generally allergic reactions are used in the same sense as type I (Roitt I, Brostoff J, Male D. Immunology 6th eds. U.S.A. Mosby. 2001:323-383).

Representative allergic diseases include atopic dermatitis, bronchial asthma, allergic rhinitis, allergic keratitis, skin urticaria, etc., and mast cells are known as important acting cells in these allergic diseases (Wills-Karp M., et al., Science, 282:2258-61, 1998; Grunig G., et al., Science, 282:2261-2263, 1998). Mast cells are cells that are widely distributed in organs throughout the body, such as skin, respiratory tract, peri-lymphatic vessels, gastrointestinal mucosa, peri-blood vessels, and brain (Ishizaka t., et al. J. Immunol. 119:1589, 1997).

The allergic reaction activates mast cells with crosslinking and aggregation of a receptor (FcRIα), resulting in the increase of a concentration of calcium ion (Ca++) in the cytoplasm, when allergens such as mites and pollen are bound to IgE, which is bound to the IgE high affinity receptor (FcεRI) on the surface of such mast cells. The increase in a concentration of calcium ion acts on various intracellular regulators such as cholesterol or cytoskeletal system of the cell membrane to cause a degranulation through a vacuolation of granules, a fusion of inter-granules and a fusion of granules and a cell membrane, simultaneously with moving the granules to the cell membrane. When being degranulated, histamine, serotonin, leukotriene, etc., which have functions such as vasodilation, hypervascular permeability, contraction or hypersecretion of bronchial smooth muscle, are released, and also NCF, ECF, PAF etc., which act as a chemotactic factor for inflammatory cells such as eosinophils or neutrophils, etc., are released together, causing an allergic reaction (Ennis M, et al., Br J Pharmacol 70:329-334, 1980; Metcalfe DD, et al., Crit Rev Immunol 3:23-74, 1981; Miyajima I, et al., J Clin Invest 99:901-914, 1997; Church MK, et al., J Allergy Clin Immunol 99:155-160, 1997; Jones DA, et al., J Biol Chem 268:9049-9054, 1993).

Bronchial asthma is an allergic disease characterized by reversible airway obstruction, airway hypersensitivity (increased airway resistance), mucus hypersecretion, and high levels of IgE in the serum. When TH2 (T helper 2) type immune cells stimulated by various antigens inhaled into the airways produce IL-4, 5, 13, etc., it is recognized as a chronic inflammatory disease because inflammatory cells such as T cells, eosinophils, mast cells, etc., are proliferated, differentiated, and activated, and thereby move to the tissues of the airways and around the airways, and infiltrates into the tissues (J Clin Invest, 111: 291-297, 2003; N Engl J. Med. 2001; 44(5): 350-62; Toshio Hirano. Cytokine molecular biology. World Science. 2002). Therefore, in the development of a therapeutic agent for asthma, suppression of the invasion of inflammatory cells such as eosinophils, etc. is one of the important targets.

Chronic obstructive pulmonary disease (COPD) is a chronic airway disease showing cough, sputum, respiratory distress, decreased expiratory flow, and impaired gas exchange, and it is predicted that COPD will be a third cause of death for humanity in 2020 (Am J Respir Crit Care Med, 2013, 187:347-365; Am J Respir Crit Care Med, 2009, 180:396-406).

COPD is developed by a variety of causes such as smoking, air pollution, chemicals, occupational factors, and genetic predisposition, etc., and smoking among those is considered as the main cause, and in fact, more than 80% of COPD patients have been found to be smokers (Biol Pharm Bull, 2012, 35:1752-1760). The pathogenesis of COPD involves inflammation, activation of proteolytic enzymes in the lung, oxidative stress, etc., and the cells involved in inflammation are mainly neutrophils, macrophages, T lymphocytes, etc. These inflammatory cells produce reactive oxygen species, various inflammatory cytokines, and several proteolytic enzymes that cause tissue damage (Korean Tuberculosis and Respiratory Society Respiratory Science Seoul: Gunja Publishing House; 2007, p 301-5; Am J Respir Crit Care Med, 1997 155, 1441-1447; Am J Physiol Lung CellMol Physiol, 2010, 298: L262-L269). In particular, neutrophils are known to play an important role in the onset of COPD by causing lung damage due to the secretion of substances such as elastase, collagenase, proteases such as myeloperoxidase (MPO), arachidonates, and reactive oxygen free radicals outside the cell, and thus, have become an important target in the development of COPD therapeutics (Am J Respir Cell Mol Biol, 2013, 48:531-539; Eur Respir J, 1998, 12:1200-1208).

Recently, it has been reported that the activation of Netosis of neutrophil extracellular traps is involved in the pathogenesis of chronic lung/respiratory diseases including COPD (PLoS One. 2014 May 15;9(5):e97784.; Respir Res. 2015 May 22;16:59.; J Immunol Res. 2017;2017:6710278; Respirology. 2016 Apr;21(3):467-75).

Currently, bronchoalveolar lavage (BAL) is used as a means to observe the course of diseases such as COPD and asthma, and in bronchoalveolar lavage fluid (BALF), inflammatory cytokines, reactive oxygen species, leukotriene, and inflammatory mediating substances such as activation complement increase, and neutrophils, which account for less than 5% of normal lungs, increase to an extent which accounts for 80% of all cells (Am J Respir Crit Care Med 154(1): 76-81, 1996).

So far, no drugs have been reported to directly improve COPD or asthma, and as a current therapeutic agent for COPD or asthma, bronchodilators (β2-agonists, anticholinergics, methylxanthines) and steroids (inhalation, oral), etc., are mainly used to reduce symptoms and complications.

### [Disclosure]

### [Technical Problem]

An object of the present disclosure is to provide a food composition for reinforcing respiratory function and alleviating respiratory diseases using extract of Paliurus ramosissimus (Lour) Poir and the like, a health functional food containing the food composition, a pharmaceutical composition for preventing or treating respiratory diseases using extract of Paliurus ramosissimus (Lour) Poir and the like, an anti-allergic food composition and a health functional food including the same. Other or specific objects of the present disclosure will be presented below.

### [Technical Solution]

As confirmed in the following Examples and Experimental Examples, the present disclosure has been completed by confirming that extracts of Paliurus ramosissimus, Rosa davurica, Picrasma quassioides, Camellia sinensis, Chrysosplenium grayanum , Reynoutria elliptica, Chaenomeles sinensis, Hydrangea serrata and/or Euonymus alatus inhibit the activation of Netosis of neutrophil extracellular traps induced by phorbol myristate acetate (PMA), inhibit the expression of IL-8, an inflammatory cytokine by CSE of NCI-H292 cells, a bronchial epithelial cell line, and inhibit the expression of MIP2, the inflammatory cytokine even in alveolar macrophages MH-S cells activated by CES and lipopolysaccharide (LPS).

In consideration of the above, in one aspect, the present disclosure discloses an anti-allergy composition comprising one or more selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata and Euonymus alatus as an effective ingredient, and in other aspects, discloses a composition for alleviating COPD, comprising one or more selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata and Euonymus alatus as an active ingredient. In the other aspect, the present disclosure discloses a composition for alleviating asthma, comprising one or more selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata and Euonymus alatus as an effective ingredient, furthermore, a composition for reinforcing respiratory function and alleviating respiratory diseases, comprising one or more selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata and Euonymus alatus as an effective ingredient, and also a composition for alleviating lung diseases accompanied by cough, sputum, respiratory distress, airway hypersensitivity, airway obstruction, mucus hypersecretion, decreased expiratory flow, and/or impaired gas exchange, comprising one or more selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata and Euonymus alatus as an effective ingredient. These lung diseases include diffuse interstitial lung disease, acute respiratory distress syndrome (ARDS), acute lung injury, etc., as well as asthma and COPD mentioned above, and in particular, include pulmonary diseases caused by fine dust, which has become a problem recently, in addition to pulmonary diseases caused by smoking, environmental contamination, genetic predisposition, etc., regardless of the cause of occurrence. Fine dust containing various components such as carbon components such as soot and organic carbon in living organisms, ionic components such as chlorine, nitric acid, ammonium, sodium, calcium, metal components such as lead, arsenic, and mercury, and polycyclic aromatic hydrocarbons such as benzopyrene, is known to cause various lung diseases such as asthma and COPD by depositing on the upper airway, bronchi, small airways, alveoli, etc. (J Korean Med Assoc, 2014; 57: 763-768).

In the present specification, the term "extract" refer to extract obtained by leaching stems, leaves, fruits, flowers, roots, and a mixture thereof of plants to be extracted, using water, lower alcohols having 1 to 4 carbon atoms (methanol, ethanol, butanol, etc.), methylene chloride, ethylene, acetone, hexane, ether, chloroform, ethyl acetate, butyl acetate, N,N-dimethylformamide (DMF), dimethyl sulfoxide (DMSO), 1,3-butylene glycol, propylene glycol, or a mixed solvent thereof, an extract obtained by using a supercritical extraction solvent such as carbon dioxide, pentane, or a fraction obtained by fractionating the extract, and as an extraction method, any method, such as macerating, refluxing, heating, ultrasonic radiation, and supercritical extraction may be applied considering the polarity, the degree of extraction, and the degree of preservation of the active substance. In the case of a fractionated extract, it means including the fraction obtained by suspending the extract in a specific solvent and mixing and holding with a solvent having a different polarity, adsorbing the crude extract on a column filled with silica gel, etc., and then using a hydrophobic solvent, a hydrophilic solvent, or a mixed solvent thereof as a mobile phase. In addition, the meaning of the extract includes a concentrated liquid extract or a solid extract from which the extraction solvent has been removed by a method such as freeze drying, vacuum drying, hot air drying, spray drying, etc. Preferably, it means an extract obtained by using water, ethanol or a mixed solvent thereof as an extraction solvent, more preferably an extract obtained by using a mixed solvent of water and ethanol as an extraction solvent.

In the present specification, the term "effective ingredient" refers to an ingredient capable of exhibiting a desired activity by itself, or capable of exhibiting activity together with a carrier that is not itself active.

In the present disclosure, the term "reinforcing respiratory function" refers to any action that maintains the original functions of the respiratory organs such as nasal cavity, pharynx, larynx, trachea, bronchi and lungs in a healthy state or improves the function of the respiratory tract, which has been deteriorated due to symptoms according to smoking, fine dust, activation of neutrophil Netosis, or other respiratory diseases, etc., to its original healthy state.

In addition, in the present disclosure, the "respiratory disease" refers to a disease occurring in the respiratory tract of an individual such as the nasal cavity, pharynx, larynx, trachea, bronchi and lungs, and specifically, the respiratory disease may mean a respiratory disease caused by smoking or fine dust; or a lung disease accompanied by Netosis but is not particularly limited thereto. More specifically, the respiratory disease may mean a pulmonary disease accompanied by symptoms of sputum, respiratory distress, airway irritability, airway obstruction, mucus hypersecretion, decrease in expiratory flow rate and/or impaired gas exchange, and more specifically, one or more selected from the group consisting of asthma, chronic obstructive pulmonary disease (COPD), tracheitis, bronchitis, diffuse interstitial lung disease, acute respiratory distress syndrome (ARDS), acute lung injury, cystic fibrosis, bronchiolitis, influenza virus infection, pneumonia, tuberculosis, and transfusion-related acute lung injury, and most specifically, may mean asthma or COPD. Meanwhile, it has been recently reported that the activation of Netosis of neutrophil extracellular traps is involved in the pathogenesis of chronic respiratory diseases including COPD.

In the composition of the present disclosure, the effective ingredient may be included in any amount (effective amount) depending on a specific use, formulation, etc., as long as it represents anti-inflammatory activity, anti-allergic activity, asthma-improving activity, COPD-improving activity, other pulmonary disease-improving activity, respiratory function reinforcement, and respiratory disease-improving activity, and a conventional effective amount will be determined within the range of 0.001% by weight to 20.0% by weight based on the total weight of the composition. Here, the term "effective amount" refers to the amount of the effective ingredient contained in the composition of the present disclosure that can represent the intended functional and pharmacological effects such as anti-inflammatory effect, anti-allergic effect, etc., when the composition of the present disclosure is administered to a mammal, preferably a human, to be applied, during the administration period according to the recommendations of a medical professional, etc. Such effective amounts may be determined empirically within the ordinary skill of a person skilled in the art.

Subjects to which the composition of the present disclosure may be applied (prescribed) are mammals and humans, particularly preferably humans.

The composition of the present disclosure, in addition to the effective ingredient, may further contain any compound or natural extract, which has already been verified for safety in the art, and is known to have the corresponding activity, in order to increase and reinforce the anti-inflammatory effect, anti-allergic effect, and an effect of alleviating asthma.

These compounds or extracts may include compounds or extracts listed in the pharmacopoeia of each country ("Korea Pharmacopoeia" in Korea), health functional food code of each country ("health functional food criteria and standards" notified by the Ministry of Food and Drug Safety in Korea), compounds or extracts that have been licensed as items in accordance with the laws of each country governing the manufacture and sale of pharmaceuticals ("Pharmaceutical Affairs Act" in Korea), or compounds or extracts whose functionality is recognized under the laws of each country governing the manufacture and sale of health functional food (┌The Act on Health Functional Foods┘ in Korea) .

For example, dimethylsulfonylmethane (MSM), N-acetylglucosamine, etc., of which anti-inflammatory ("arthritis improvement") functions are recognized, according to ┌Law regarding Health Functional Foods Act┘, Enterococcus faecalis heat-treated dry powder, which has been recognized for its anti-allergic ("relieving hypersensitive immune response") functionality, complexes of guava leaf extract, etc., complexes of sagebrush extract, lobule extract, picaopreto powder, etc., PLAG (1-palmitoyl-2-linoleoyl-3-acetyl-rac-glycerol), etc., correspond to the compound or extract, and are not particularly limited thereto.

One or more of these compounds or natural extract may be included in the composition of the present disclosure together with the effective ingredient.

In a specific aspect, the composition of the present disclosure can be considered as a food composition. In addition, the food of the present disclosure may include health functional food. In the present disclosure, the term "health functional food" refers to a food manufactured and processed in the form such as tablets, capsules, powders, granules, liquids and pills using raw materials or ingredients having useful functions for the human body. Here, the term "functionality" refers to obtaining useful effects for health purposes such as controlling nutrients or physiological effects on the structure and function of the human body. The health functional food of the present disclosure may be prepared by a method commonly used in the art, and at the time of manufacture, may be prepared by adding raw materials and ingredients commonly added in the art. In addition, the formulation of the health functional food may be prepared without limitation as long as it is a formulation recognized as a health functional food. The food composition of the present disclosure may be prepared in various forms of formulation, and unlike general drugs, it has the advantage of not having side effects that may occur when taking the drug for a long time using food as a raw material, and health functional foods may be ingested as an adjuvant to enhance the anti-allergic effect, the effect of alleviating asthma or the effect of alleviating COPD, and further reinforcing the respiratory function and alleviating the respiratory disease.

The food composition of the present disclosure may be prepared in any form, for example, beverages such as tea, juice, carbonated beverages, ionized beverages, processed milk such as milk and yogurt, foods such as gums, rice cakes, Korean sweets, bread, crackers, and noodles, health functional food preparations such as tablets, capsules, pills, granules, liquids, powders, flakes, pastes, syrups, gels, jelly, and bars.

In addition, the food composition of the present disclosure may be classified as a product as long as it conforms to the enforcement regulations at the time of manufacture and distribution in terms of legal and functional classification. For example, it may be a health functional food according to the Korean ┌Health Functional Food Act┘, or crackers, bean, tea, beverages, special purpose food, etc., according to the food code (┌Food criteria and standards┘ notified by the Ministry of Food and Drug Safety) of the Korean ┌Food Sanitation Act┘.

The food composition of the present disclosure may contain food additives in addition to the effective ingredients. Food additives may generally be understood as substances that are added to food and mixed or infiltrated in manufacturing, processing, or preserving food and since they are consumed daily and for a long time with food, their safety should be ensured. In the Food Additive Code (┌Food Sanitation Act┘ in Korea) governing the manufacture and distribution of food, food additives with guaranteed safety are limited in terms of ingredients or functions. In the Korean Food Additives Code (┌Food Additive Standards and Specification┘ notified by the Ministry of Food and Drug Safety), food additives are defined by being classified into chemical synthetic products, natural additives, and mixed preparations in terms of ingredients, and these food additives are categorized into sweeteners, flavoring agents, preservatives, emulsifiers, acidulants, thickeners, etc., in terms of function.

Sweeteners are used to give foods an appropriate sweet taste, and natural or synthetic sweeteners may be used. It is preferable to use a natural sweetener, and examples of the natural sweetener include sugar sweeteners such as corn syrup solids, honey, sucrose, fructose, lactose, and maltose.

A flavoring agent may be used to enhance taste or flavor, and both a natural flavoring agent and a synthetic flavoring agent may be used. Preferably, it is the case of using natural flavoring agents. In the case of using the natural flavoring agent, the purpose of nutrient enhancement may be combined in addition to flavor. As the natural flavoring agent, it may be obtained from apples, lemons, tangerines, grapes, strawberries, peaches, etc., or may be obtained from green tea leaves, Polygonatum, large leaves, cinnamon, chrysanthemum leaves, jasmine, and the like. In addition, those obtained from ginseng (red ginseng), bamboo shoots, aloe vera, ginkgo, etc., may be used. The natural flavoring agent may be a liquid concentrate or a solid extract. In some cases, a synthetic flavoring agent may be used, and esters, alcohols, aldehydes, terpenes, etc., may be used as the synthetic flavoring agents.

As a preservative, calcium sorbate, sodium sorbate, potassium sorbate, calcium benzoate, sodium benzoate, potassium benzoate, EDTA (ethylenediaminetetraacetic acid), etc., may be used, and as an emulsifier, acacia gum, carboxymethylcellulose, xanthan gum, pectin, etc., may be used, and as the acidulant, arithmetic, malic acid, fumaric acid, adipic acid, phosphoric acid, gluconic acid, tartaric acid, ascorbic acid, acetic acid, phosphoric acid, etc., may be used. The acidulant may be added so that the food composition has an appropriate acidity for the purpose of suppressing the growth of microorganisms in addition to the purpose of enhancing taste.

As the thickening agent, a suspending agent, a settling agent, a gel-forming agent, a swelling agent, etc., may be used.

In addition to the food additives described above, the food composition of the present disclosure may include biological active substances or minerals known in the art for the purpose of supplementing and reinforcing functionality and nutritional properties and ensuring stability as a food additive.

Such physiologically active substances may be catechins, vitamins such as vitamin B1, vitamin C, vitamin E, and vitamin B12, tocopherol, dibenzoyl thiamine, etc., contained in green tea, and minerals may be calcium preparations such as calcium citrate, magnesium preparations such as magnesium stearate, iron preparations such as iron citrate, chromium chloride, potassium iodide, selenium, germanium, vanadium, zinc. etc.

In the food composition of the present disclosure, the food additive described above may be included in an appropriate amount to achieve the purpose of addition according to a product type.

Regarding other food additives that may be included in the food composition of the present disclosure, reference may be made to the food code of each country or the food additive code.

The composition of the present disclosure may be understood as a pharmaceutical composition in other specific embodiments.

The pharmaceutical composition of the present disclosure may be prepared in an oral formulation or parenteral formulation according to an administration route by a conventional method known in the art, including a pharmaceutically acceptable carrier in addition to the effective ingredient. Here, the route of administration may be any suitable route including a local route, an oral route, an intravenous route, an intramuscular route, and direct absorption through mucosal tissue, and two or more routes may be used in combination. An example of a combination of two or more routes is a case in which two or more formulations of drugs are combined according to the route of administration, for example, when one drug is firstly administered by an intravenous route and the other drug is secondly administered by a topical route.

Pharmaceutically acceptable carriers are well known in the art according to the route of administration or formulation, and specifically, reference may be made to the pharmacopoeia of each country, including the "Korean Pharmacopoeia."

When the pharmaceutical composition of the present disclosure is prepared in an oral dosage form, a formulation such as powders, granules, tablets, pills, dragees, capsules, solutions, gels, syrups, suspensions, wafers together with a suitable carrier may be prepared according to a method known in the art. Examples of suitable carriers at this time include sugars such as lactose, glucose, sucrose, dextrose, sorbitol, mannitol, and xylitol, starches such as corn starch, potato starch, and wheat starch, celluloses such as cellulose, methylcellulose, ethyl cellulose, sodium carboxymethylcellulose, hydroxypropylmethylcellulose, polyvinyl pyrrolidone, water, methylhydroxybenzoate, propylhydroxybenzoate, magnesium stearate, mineral oil, malt, gelatin, talc, polyol, vegetable oil, ethanol, glycerol, etc. In the case of formulation, appropriate binders, lubricants, disintegrants, colorants, and diluents may be included, if necessary. Examples of suitable binders may include starch, magnesium aluminum silicate, starch paste, gelatin, methylcellulose, sodium carboxymethylcellulose, polyvinylpyrrolidone, glucose, corn sweetener, sodium alginate, polyethylene glycol, wax, etc., and examples of lubricants may include sodium oleate, sodium stearate, magnesium stearate, sodium benzoate, sodium acetate, sodium chloride, silica, talcum, stearic acid, magnesium salts and calcium salts thereof, polyethylene glycol, etc., and examples of disintegrating agents may include starch, methyl cellulose, agar, bentonite, xanthan gum, starch, alginic acid, or a sodium salt thereof. Moreover, examples of diluents may include lactose, dextrose, sucrose, mannitol, sorbitol, cellulose, glycine, etc.

When the pharmaceutical composition of the present disclosure is prepared in a parenteral formulation, it may be formulated in the form of an injection, a transdermal administration, a nasal inhalation, and a suppository together with a suitable carrier according to a method known in the art. When formulated as an injection, an aqueous isotonic solution or suspension may be used as a suitable carrier, and specifically, triethanol amine-containing phosphate buffered saline (PBS), sterile water for injection, or an isotonic solution such as 5% dextrose, etc., may be used. When the pharmaceutical composition of the present disclosure is formulated in a transdermal dosage form, it may be formulated in the form of an ointment, cream, lotion, gel, external solution, pasta, liniment, air roll, etc. In the case of nasal inhalants, suitable propellants such as dichlorofluoromethane, trichlorofluoromethane, dichlorotetrafluoroethane, carbon dioxide, etc., may be used to form an aerosol spray, and when the pharmaceutical composition of the present disclosure formulated as a suppository, witepsol, tween 61, polyethylene glycols, cacao butter, laurin butter, polyoxyethylene sorbitan fatty acid esters, polyoxyethylene stearates, sorbitan fatty acid esters, etc., may be used as the carrier.

The specific formulation of pharmaceutical compositions is known in the art, and for example, Remington's Pharmaceutical Sciences (19th ed., 1995), etc., may be referred to. This document is considered as part of this specification.

The preferred dosage of the pharmaceutical composition of the present disclosure may be in the range of 0.001 mg/kg to 10 g/kg per day, preferably 0.001 mg/kg to 1 g/kg, depending on the patient's condition, weight, sex, age, patient's severity, and route of administration. Administration may be made once a day or divided into several times. Such dosage should not be construed as limiting the scope of the invention in any aspect.

### [Advantageous Effects]

As described above, according to the present disclosure, it may provide a composition for alleviating allergy, asthma or COPD using extracts of Paliurus ramosissimus, Rosa davurica, Picrasma quassioides, Camellia sinensis, Chrysosplenium grayanum, Reynoutria elliptica, Chaenomeles sinensis, Hydrangea serrata and/or Euonymus alatus. The composition of the present disclosure is commercialized as a food, in particular a health functional food or a drug, and may be usefully used for anti-allergic effect, asthma improvement effect or COPD improvement effect, and may be further usefully used for reinforcing respiratory function and alleviating respiratory disease, other lung diseases accompanied by symptoms such as coughing, sputum, respiratory distress, airway irritability, airway obstruction, and mucus hypersecretion, etc., for example, diffuse interstitial lung disease, acute respiratory distress syndrome (ARDS), and acute lung injury, etc.

### [Description of Drawings]

FIG. 1 shows the results of evaluating the Netosis level (%) when treated with each concentration of extract of Picrasma quassioides for HL-60 cells.
FIG. 2 shows the results of evaluating the Netosis level (%) when treated with extract of Camellia sinensis for HL-60 cells.
FIG. 3 shows the results of evaluating the Netosis level (%) when treated with extract of Reynoutria elliptica for HL-60 cells.
FIG. 4 shows the results of evaluating the Netosis level (%) when treated with extract of Chaenomeles sinensis (branch, leaf) for HL-60 cells.
FIG. 5 shows the results of evaluating the Netosis level (%) when treated with extract of Euonymus alatus for HL-60 cells.
FIG. 6 shows the results of evaluating the activity of IL-8 inhibition when treated with extract of Paliurus ramosissimus for H292 cells.
FIG. 7 shows the results of evaluating the activity of IL-8 inhibition when treated with extract of Picrasma quassioides for H292 cells.
FIG. 8 shows the results of evaluating the activity of IL-8 inhibition when treated with extract of Camellia sinensis for H292 cells.
FIG. 9 shows the results of evaluating the activity of IL-8 inhibition when treated with extract of Chrysosplenium grayanum for H292 cells.
FIG. 10 shows the results of evaluating the activity of IL-8 inhibition when treated with extract of Reynoutria elliptica for H292 cells.
FIG. 11 and 12 show the results of evaluating the Netosis % when treated with extract of branch and leaf, and extract of fruit of Chaenomeles sinensis for H292 cells.
FIG. 13 and 14 show the results of evaluating the activity of IL-8 inhibition when treated with extract of leaf and extract of branch of Euonymus alatus for H292 cells.
FIG. 15 shows the results of evaluating the activity of MIP-2 inhibition when treated with extract of Paliurus ramosissimus for MH-S cells.
FIG. 16 shows the results of evaluating the activity of MIP-2 inhibition when treated with extract of Rosa davurica for MH-S cells.
FIG. 17 shows the results of evaluating the activity of MIP-2 inhibition when treated with extract of Picrasma quassioides for MH-S cells.
FIG. 18 shows the results of evaluating the activity of MIP-2 inhibition when treated with extract of Camellia sinensis for MH-S cells.
FIG. 19 shows the results of evaluating the activity of MIP-2 inhibition when treated with extract of Chrysosplenium grayanum for MH-S cells.
FIG. 20 shows the results of evaluating the activity of MIP-2 inhibition when treated with extract of fruits of Chaenomeles sinensis for MH-S cells.
FIG. 21 shows the results of evaluating the activity of MIP-2 inhibition when treated with extract of Euonymus alatus for MH-S cells.
FIGS. 22 to 30 show the total number of cells counted from bronchoalveolar lavage fluid (BALF) after administering extracts of Paliurus ramosissimus, Rosa davurica, Picrasma quassioides, Camellia sinensis, Reynoutria elliptica, branch/leaf of Chaenomeles sinensis, fruits of Chaenomeles sinensis, and Euonymus alatus for the mouse model of PPE/CSE-induced chronic obstructive pulmonary disease (or respiratory disease) in order, respectively.
FIG. 31 shows the results of analyzing an immune cell in BALF via Diff-Quick staining after administering extract of Paliurus ramosissimus for the mouse model of PPE/CSE-induced respiratory disease.
FIG. 32 shows the results of analyzing an immune cell in BALF via Diff-Quick staining after administering extract of Rosa davurica for the mouse model of PPE/CSE-induced respiratory disease.
FIG. 33 shows the results of analyzing an immune cell in BALF via Diff-Quick staining after administering extract of Picrasma quassioides for the mouse model of PPE/CSE-induced respiratory disease.
FIGS. 34 and 35 show the results of analyzing an immune cell in BALF via Diff-Quick staining after administering extract of Camellia sinensis for the mouse model of PPE/CSE-induced respiratory disease.
FIG. 36 shows the results of analyzing an immune cell in BALF via Diff-Quick staining after administering extract of Reynoutria elliptica for the mouse model of PPE/CSE-induced respiratory disease.
FIGS. 37 and 38 show the results of analyzing an immune cell in BALF via Diff-Quick staining after administering extracts of branch and leaf of Chaenomeles sinensis, and extract of fruits of Chaenomeles sinensis for the mouse model of PPE/CSE-induced respiratory disease.
FIG. 39 shows the results of analyzing an immune cell in BALF via Diff-Quick staining after administering extract of Hydrangea serrata for the mouse model of PPE/CSE-induced respiratory disease.
FIGS. 40 and 41 show the results of analyzing an immune cell in BALF via Diff-Quick staining after administering extract of Euonymus alatus for the mouse model of PPE/CSE-induced respiratory disease.
FIGS. 42 to 44 show the results of staining the lung tissue extracted from the mouse model to which extracts of Camellia sinensis, fruits of Chaenomeles sinensis, and Euonymus alatus are administered in order, respectively.
FIGS. 45 to 50 show the results of analyzing cytokine in BALF for the mouse model to which extracts of Picrasma quassioides, Camellia sinensis, Reynoutria elliptica, Chaenomeles sinensis, Hydrangea serrata, and Euonymus alatus are administered in order, respectively.
FIG. 51 shows the results of analyzing cytokine (KC, MCP-1, MDC, TARC, GM-CSF, MIP-2, IL-6, TNF-alpha, IL-1beta, INF-gamma, IL-10, and IL-17) from the lung tissue of the mouse model to which extract of Euonymus alatus is administered.

### [DETAILED DESCRIPTION OF THE EMBODIMENTS]

Hereinafter, the present disclosure will be described with reference to Examples and Experimental Examples. However, the scope of the present disclosure is not limited to these Examples and Experimental Examples.

### [Example] Preparation of sample

Extracts of Paliurus ramosissimus (extraction site: leaf), Rosa davurica (extraction site: leaf, stem), Picrasma quassioides (extraction site: leaf), Camellia sinensis (extraction site: leaf, stem), Chrysosplenium grayanum (extraction site: outpost), Reynoutria elliptica (extraction site: root), Hydrangea serrata (extraction site: above-ground), Euonymus alatus (extraction site: leaf, branch), and Chaenomeles sinensis (extraction site: leaf, branch, fruit) were prepared to confirm the activity of alleviating the lung diseases. The extract was obtained in the form of a powder by adding 10 times weight of 70% ethanol to the dry powder of each material, respectively, extracting twice at 50°C for 6 hours each, filtering, concentration under reduced pressure, and freeze-drying.

### [Experimental Example] Activity of alleviating the lung disease

### Experimental Example 1: Activity of Netosis inhibition in neutrophil cell

### 1-1: Extract of Paliurus ramosissimus, Rosa davurica, Chrysosplenium grayanum, and Hydrangea serrata

It has been known that the neutrophil extracellular traps (Netosis) are involved in the pathogenesis of patients with chronic lung/respiratory diseases including the chronic obstructive lung disease (PLoS One. 2014 May 15;9(5):e97784.; Respir Res. 2015 May 22;16:59.; J Immunol Res. 2017;2017:6710278; Respirology. 2016 Apr;21(3):467-75.)). Accordingly, the Netosis activity of each extract was confirmed in a model wherein neutrophil cells were treated with phorbol myristate acetate (PMA).

In order to measure the Netosis activity of neutrophils, Human promyelocytic leukemia (HL-60) cells obtained from the Korean Cell Line Bank were used in the experiment. HL-60 cells were treated with 2×10⁵ cells/mL in RPMI (10% FBS, 100 U/mL penicillin, 100 mg/mL streptomycin) medium treated with 1% DMSO and incubated in a cell incubator (37°C, 5% CO₂) for 5 days and differentiated to give a mature neutrophil phenotype. After 5 days, after centrifugation at 1,300 rpm for 5 minutes, it was treated with 10 mL of HBSS (with Mg+, Ca+, Hanks' balanced salt solution), followed by centrifugation at 1,300 rpm for 5 minutes to wash. Next, it was diluted in HBSS at 2×10⁶ cells/mL, was dispensed 50 µL each, and was treated with 50 µL each of the sample of the above example which was prepared at each concentration, and it was incubated for 3 hours after treatment in each well of 100 µL of PMA (in HBSS) which was prepared at 200 nM (final PMA concentration 100 nM). After 3 hours, it was treated with 500 nM of Cytox green and measured at a wavelength of 485/520 nm (excitation/emission maxima) using a fluorescent plate reader. Here, PMA is generally used to elicit the Netosis activity of neutrophil cells.

The Netosis inhibitory activity of the extracts of Paliurus ramosissimus, Chrysosplenium grayanum, and Hydrangea serrata compared with control group (experimental control group) treated with PMA, was measured as a percentage, and the average values thereof were shown as % in Tables 1 to 3, respectively (n>3). On the other hand, it was confirmed that extract of Rosa davurica exhibited an inhibitory activity of about 71.1% at a concentration of 50 *µg*/ml.

**[Table 1]**

| Concentration of the treatment with Paliurus ramosissimus | 6.25 *µg*/ml | 12.5 *µg*/ml | 25 *µg*/ml | 50 *µg*/ml |
|---|---|---|---|---|
| Netosis inhibitory activity (%) | 97.9 | 136.8 | 143.0 | 264.3 |

**[Table 2]**

| Concentration of the treatment with Chrysosplenium grayanum | 25 µg/mL | 50 µg/mL |
|---|---|---|
| Netosis inhibitory activity (%) | 0.9 | 9.5 |

**[Table 3]**

| Concentration of the treatment with Hydrangea serrata | 12.5 µg/mL | 25 µg/mL |
|---|---|---|
| Netosis inhibitory activity (%) | 24.8 | 58.6 |

### 1-2: Picrasma quassioides, Camellia sinensis, Reynoutria elliptica, Chaenomeles sinensis, Euonymus alatus

HL-60 cells in 2×10⁵ cells/mL were seeded to RPMI (10% FBS, 100 U/mL penicillin, 100 mg/mL streptomycin) medium treated with 1% DMSO and incubated in a cell incubator (37°C, 5% CO₂) for 5 days, and differentiated to a mature neutrophil phenotype. After 5 days, it was treated with 10 mL of HBSS (with Mg⁺, Ca⁺) after centrifugation at 1,300 rpm for 5 minutes, followed by centrifugation at 1,300 rpm for 5 minutes to wash. The recovered cells were diluted in HBSS at a concentration of 1×10⁶ cells/mL, were dispensed into 96 well plates by 100 µL each, and 50 µL of samples prepared at each concentration were treated. Then, PMA prepared at 400 nM was treated in each well by 50 µL and incubated for 3 hours. After 3 hours, it was treated with Cytox green (500 nM) and fluorescence was measured at excitation/emission maxima: 485/520 nm using a fluorescent plate reader.

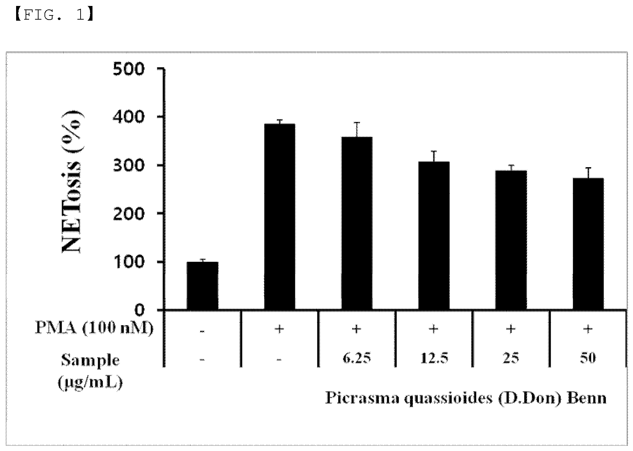

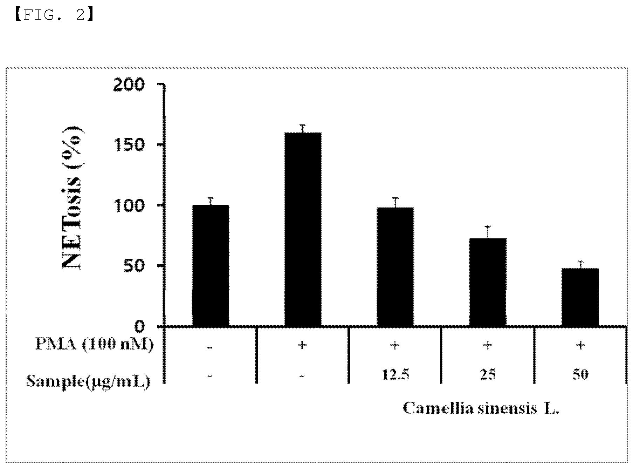

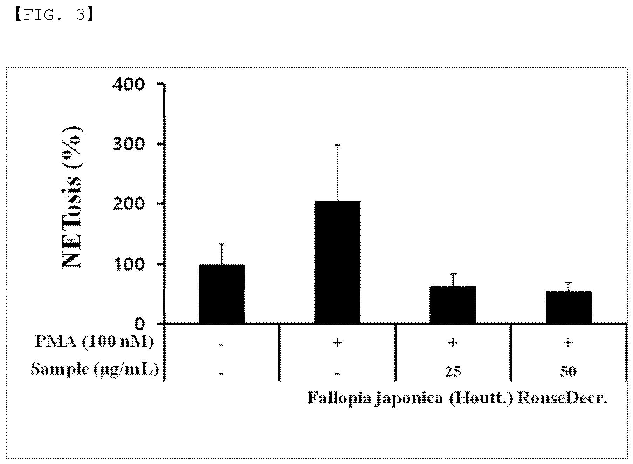

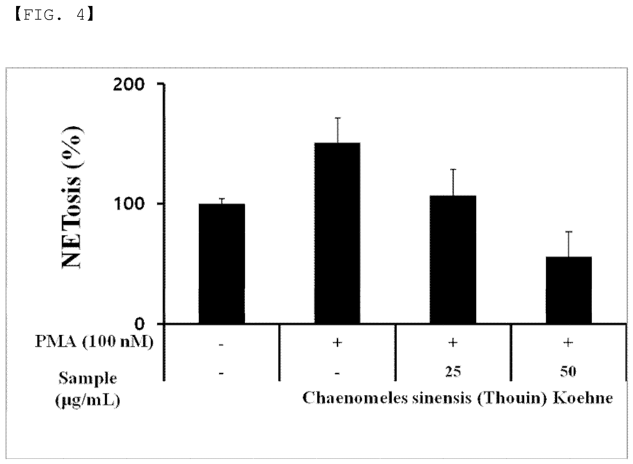

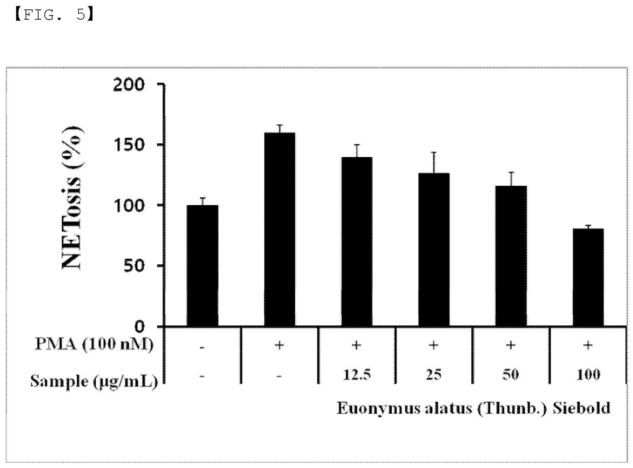

As a result, it was confirmed that the level of Netosis (%) was decreased depending on the concentration of the treated extracts of Picrasma quassioides, Camellia sinensis, Reynoutria elliptica, Chaenomeles sinensis (branch, leaf), and Euonymus alatus (FIGS. 1 to 5).

### Experimental Example 2: Evaluation of inflammatory cytokine (IL-8) Inhibitory activity using H292 cells

### 2-1: Extract of Paliurus ramosissimus, Rosa davurica, Picrasma quassioides, Camellia sinensis, Chrysosplenium grayanum, Reynoutria elliptica, Chaenomeles sinensis, and Euonymus alatus

H292 cells prepared at a concentration of 2×10⁵ cells/mL in a 24 well plate, were passaged and cultured at 500 µL/well, and when the cells are confluent to be 80% or more, the medium was exchanged with serum-free RPMI1640 medium and subject to a starvation for 24 hours. Then, using Serum-free RPMI1640 medium, the stimulus (CSE; final 2%) and each sample were diluted to reflect a desired final concentration, and then treated at 500 uL each per well. After incubation overnight, the supernatant was recovered and used for IL-8 measurement. Human IL-8 was measured by an ELISA method, and was subject to the manufacturer's (BD company) protocol for further proceeding. Here, the supernatant for IL-8 measurement was diluted 5 times and analyzed.

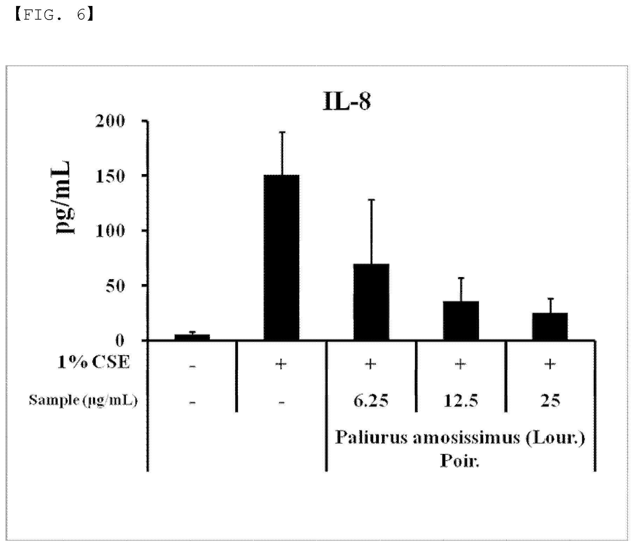

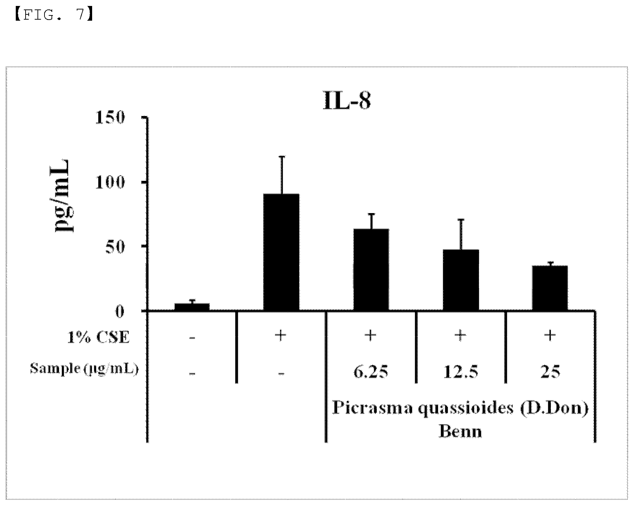

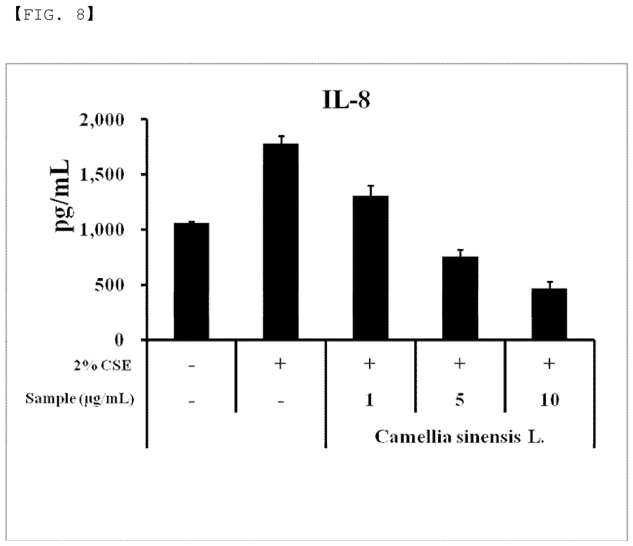

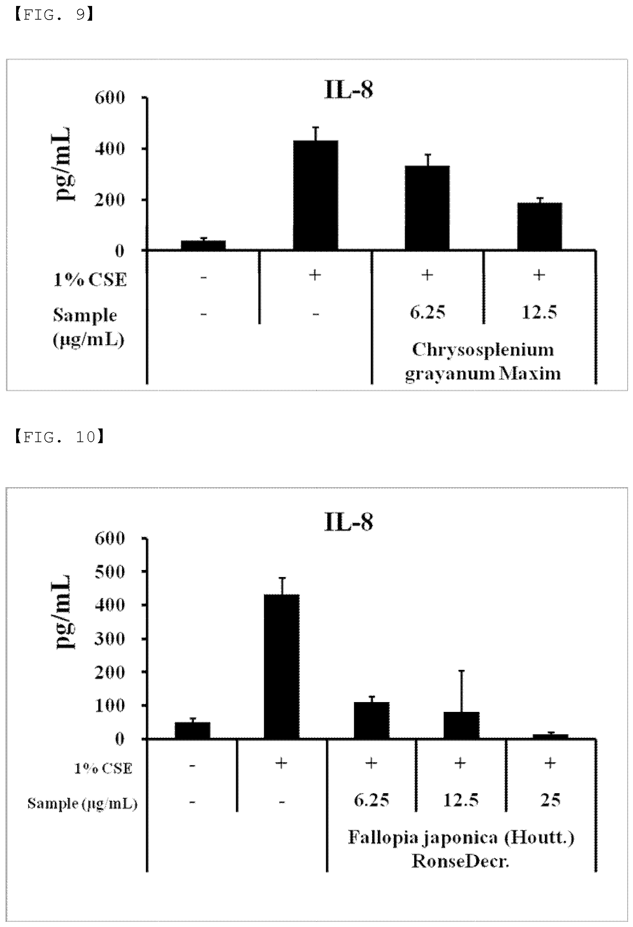

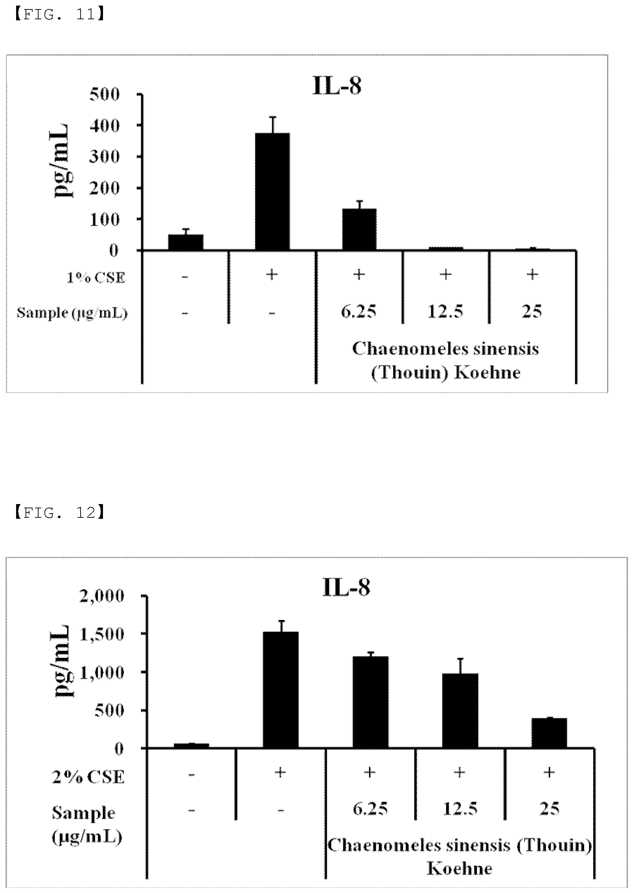

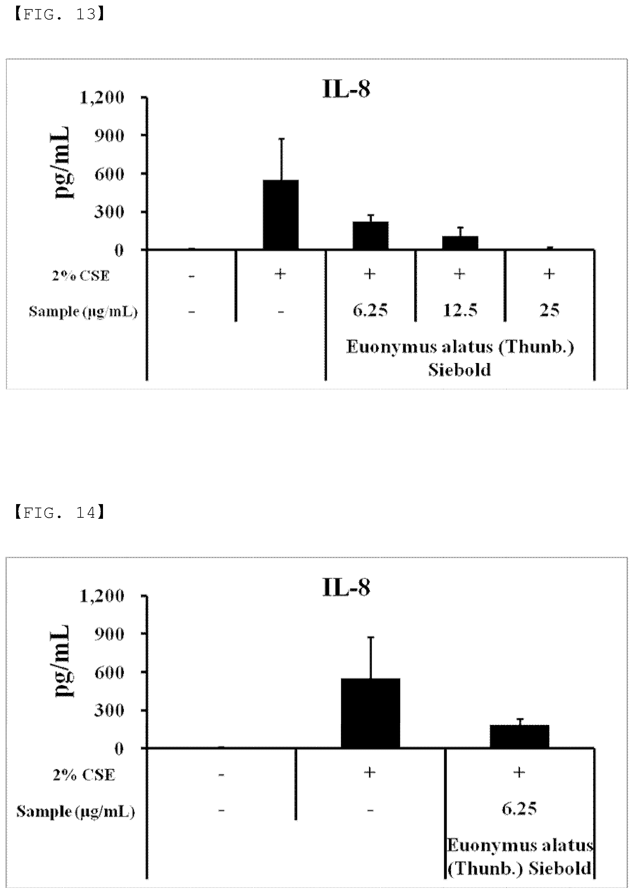

As a result, it was confirmed that the concentration of IL-8 was inhibited depending on the concentrations of the extract of the treated Paliurus ramosissimus, Picrasma quassioides, Camellia sinensis, Chrysosplenium grayanum, Reynoutria elliptica, Chaenomeles sinensis (branch/leaf, fruits), and Euonymus alatus (leaf, branch) (FIGS. 6 to 14). On the other hand, it was confirmed that extract of Rosa davurica exhibited an inhibitory activity of about 60.1% of IL-8 at a concentration of 100 µg/ml.

### 2-2: Extract of Hydrangea serrata

NCI-H292 cells, the bronchial epithelial cell line, were obtained from the Korea Cell Line Bank and used. RPMI1640 (WELGENE, cat. LM011-51) containing 10% fetal bovine serum (WELGENE, cat. S001-07) and 1% penicillin-streptomycin (WELGENE, cat. LS202-02) was used as a culture solution, and incubated in an incubator (37°C, 5% CO₂).

NCI-H292 cells at a concentration of 5×10⁴ cells/ml were dispensed at 200 µl each per well of a 96-well plate and cultured and when about 80% was full, the medium was replaced with serum-free RPMI medium. One day later, it was again exchanged with CSE (final 1%) and 200 µl of serum-free RPMI medium to which the sample was added, and after 24 hours, 150 µl of the supernatant was recovered and used for cytokine measurement (ELISA).

One day before the experiment, a capture antibody against IL-8 was diluted to an appropriate concentration in PBS, and 100 µl each per well of a 96 well immune plate (SPL) was dispensed and left overnight. Thereafter, after washing three times using a washing solution of 350 µl each per well, 300 µl of a diluted solution (Reagent Diluent) was added, and left at room temperature for 1 hour, followed by washing twice. Thereafter, standard and supernatant samples were diluted to an appropriate concentration in the diluted solution, treated with 100 µl each per well, incubated for 2 hours at room temperature, and washed twice. Using the diluent solution, the IL-8 detection antibody was diluted to an appropriate concentration, treated with 100 µl each per well, and incubated at room temperature for 2 hours. Later, after washing twice, 100 µl each of a diluted solution containing streptavidin-HRP was treated, and incubated for 20 minutes at room temperature, followed by washing twice. Finally, 50 µl of a substrate solution was treated per well, the plate was tapped lightly, and absorbance was measured at 450 nm wavelength using a microplate reader. As a negative control group, a group that was not treated with any substance was used, and a group treated with CSE (final concentration 1%) was used as a sham control, and the results of inhibiting the production of IL-8 as measured according to the above was calculated as an average % compared to the experimental control group.

As a result, extract of Hydrangea serrata showed an inhibitory activity of 83.8% of IL-8 at a concentration of 100 µg/mL.

### Experimental Example 3: Evaluation of MIP-2 inhibitory activity using MH-S cells

### 3-1: Extract of Paliurus ramosissimus, Rosa davurica, Picrasma quassioides, Camellia sinensis, Chrysosplenium grayanum, Reynoutria elliptica, Chaenomeles sinensis, and Euonymus alatus

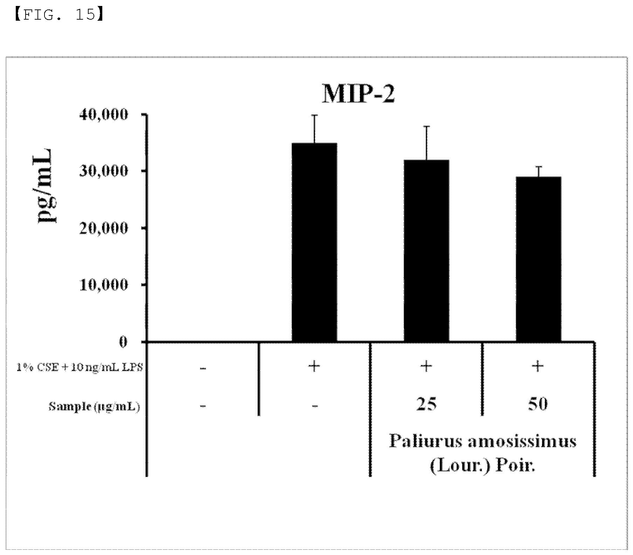

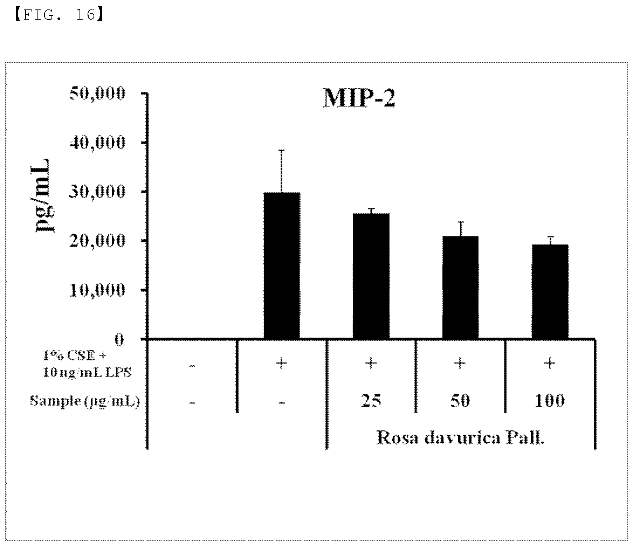

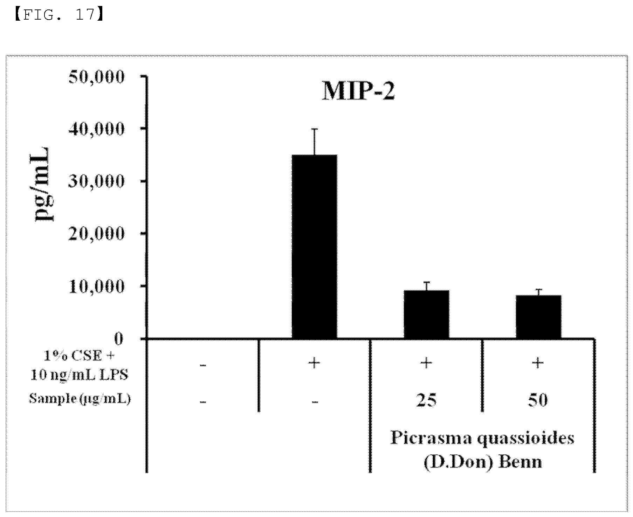

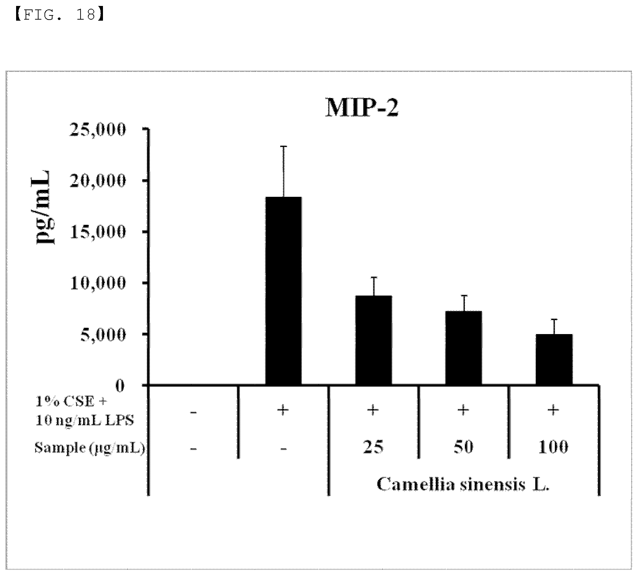

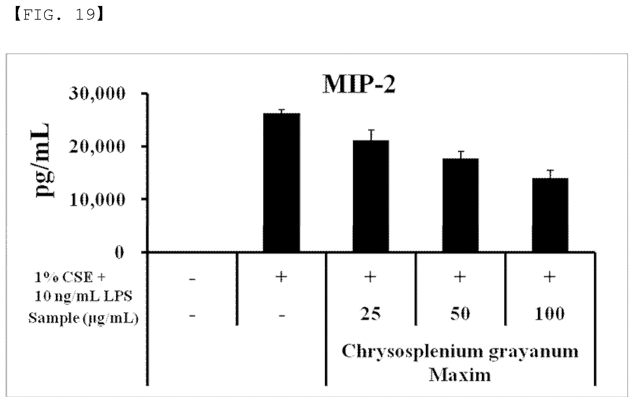

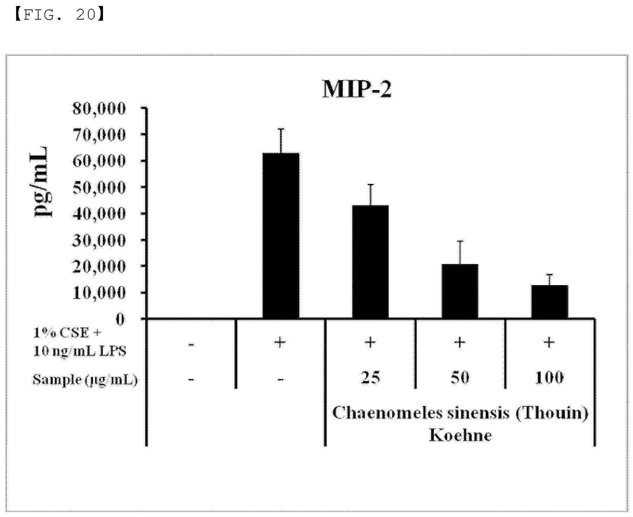

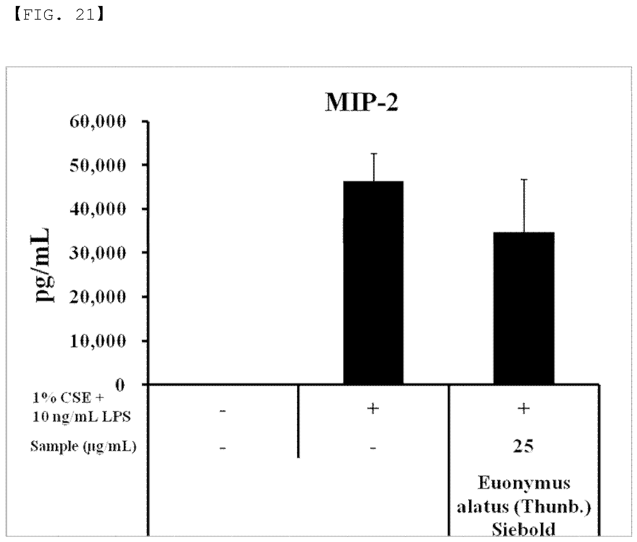

The MH-S cell line was purchased from ATCC, and the medium composition used for cultivation was RMPI-1640 (WELGENE, cat. LM 011-01), 10% FBS (WELGENE, cat. S 001-07), 1% penicillin-streptomycin (WELGENE, cat. LS 202-02), 14.3 mM 2-mercptoethanol (SIGMA). MH-S cells were seeded to a cell culture 96 well plate (SPL, cat. 30096) at a concentration of 5x10⁴/well and cultured for 24 hours. Thereafter, stimulants (CSE; final 1% and LPS; final 10 ng/mL) and each sample were diluted to reflect the desired final concentration with MH-S cell culture medium and treated at 250 uL each per well. After incubation for 24 hours, the supernatant was recovered and used for MIP-2 measurement. MIP-2 was measured by the ELISA method, and was subject to the manufacturer's (R&D, DY452) protocol for further proceeding. The measured MIP-2 expression inhibition result was calculated as an average % compared to the experimental control group (CSE-treated group) (FIGS. 15 to 21). On the other hand, it was confirmed that MIP-2 secretion was inhibited (11.5%) when extract of Reynoutria elliptica was treated at a concentration of 25 µg/ml.

### 3-2: Extract of Hydrangea serrata

MH-S cells were cultured in a cell incubator (37°C, 5% CO₂) by using RPMI-1640 medium (WELGENE, cat. LM 011-01) containing 10% fetal bovine serum (WELGENE, cat. S 001-07), 1% penicillin-streptomycin (WELGENE, cat. LS 202-02) and 0.05 mM 2-Mercaptoethanol (SIGMA).

After removing the medium present in the MH-S culture dish, it was washed twice with 5 ml of DPBS. After removing all DPBS, 10 ml each of MH-S culture medium was dispensed into the dish, and cells were collected using a scraper. Cells were dispensed to a 15 ml tube, centrifuged under conditions of 1000 rpm, 24°C, and 10 minutes to remove the supernatant, and 1 ml each of the medium was dispensed. After measuring the number of cells using a cell counter, it was diluted to 2×10⁵ cells/ml, and 250 µl each was dispensed into a 96 well plate (SPL, cat. 30096). After culturing the cells for 24 hours, it was exchanged with 250 µL of the culture medium containing 1% CSE, 10 ng/ml LPS, and each sample and cultured for an additional 24 hours. After 24 hours, the supernatant present in 96 wells was removed separately for cytokine measurement (ELISA).

Using the supernatant obtained through the above experiment, the expression level of MIP-2 in alveolar macrophages was measured by the ELISA method in the same manner as in Experimental Example 2. The result of inhibiting an expression of MIP-2 as measured was calculated as an average % compared to the experimental control group.

As a result, extract of Hydrangea serrata showed excellent inhibitory activity of 85.9% at 25 µg/mL concentration, 97.4% at 50 µg/mL concentration, and 99.5% at 100 µg/mL concentration for MIP-2 expression, respectively (Table 4).

**[Table 4]**

| Concentration of the treatment with Hydrangea serrata | 25 µg/mL | 50 µg/mL | 100 µg/mL |
|---|---|---|---|
| Inhibition of an expression of MIP-2(%) | 85.9 | 97.4 | 99.5 |

### Experimental Example 4: Measurement of BALF cell count in animal models

### 4-1: A mouse model of PPE+CSE-induced respiratory disease

BALB/C mice (male, 5 weeks old) were purchased from Orient Bio Inc., and were adapt for 1 week, and grouped with n=10. The sample treatment group was orally administered 200 mg/kg of a sample for 24 days from one week before COPD induction until before dissection, and the Naive and COPD groups were administered the same amount of PBS. At this time, a concentration of 10 mg/kg was orally administered to the positive control, Roflumilast group, daily after initiating induction. For COPD induction, 1.2 units of Porcine Pancreatic Elastase (PPE) and 100% Cigarette Smoke Extraction (CSE) were used. 1.2 unit of PPE was injected three times through intra nasal once every 7 days, and 20 µl of CSE was treated through intra nasal for 3 days from the day after PPE treatment, but after the last PPE treatment, the dissection proceeded the next day without CSE treatment. Dissection was performed after anesthesia with isoflurane using an inhalation anesthesia machine. During the experiment, feed and drinking water were provided in the form of self-catering, and were reared in an environment of 24°C and 60% humidity.

### 4-2: Total number of cells infiltrated into BALF

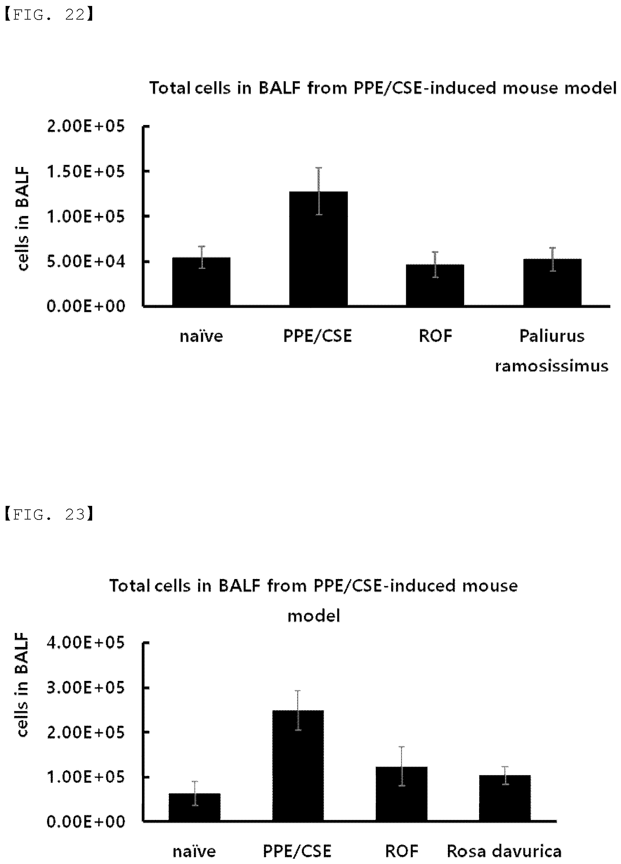

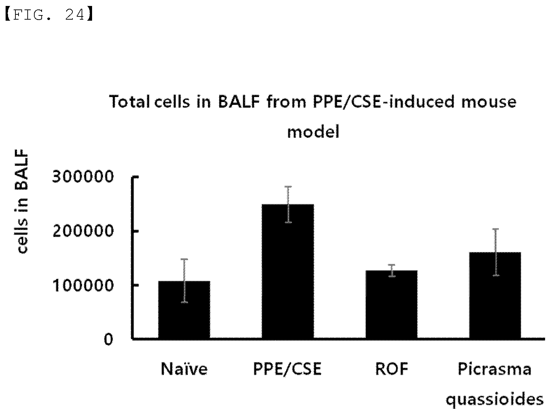

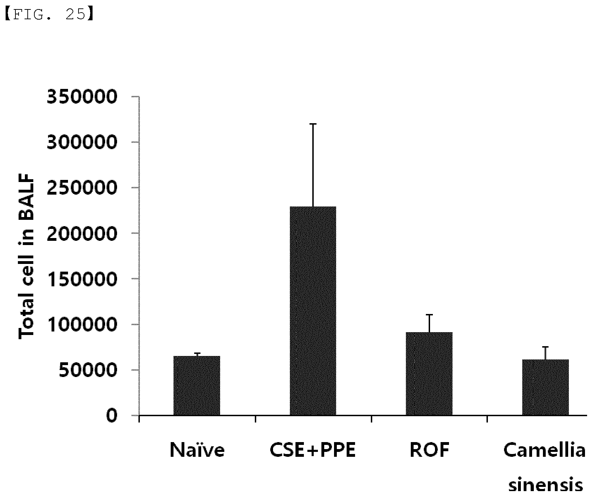

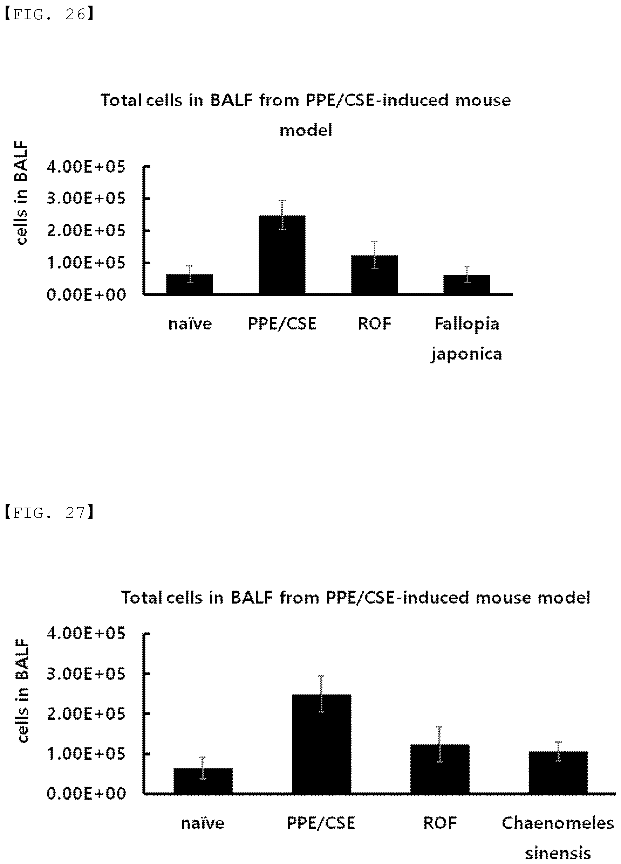

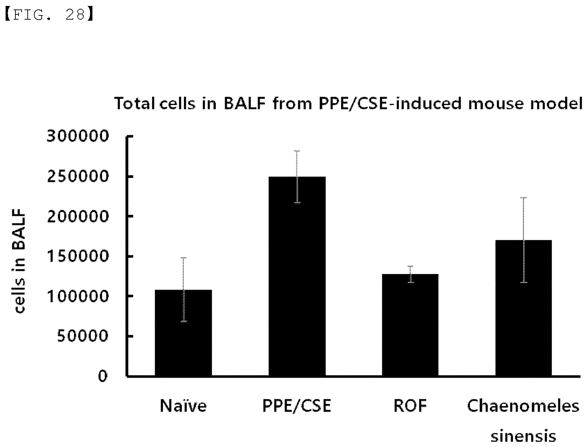

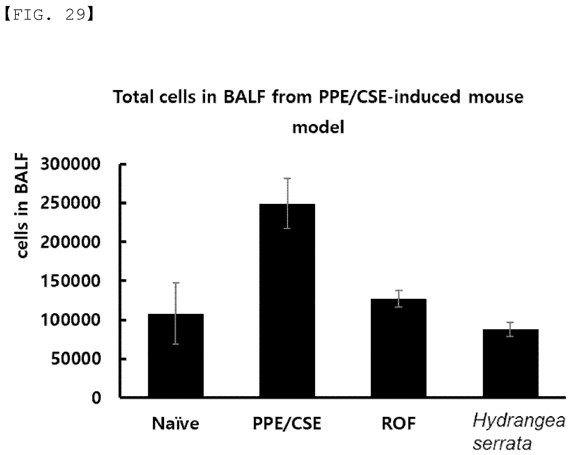

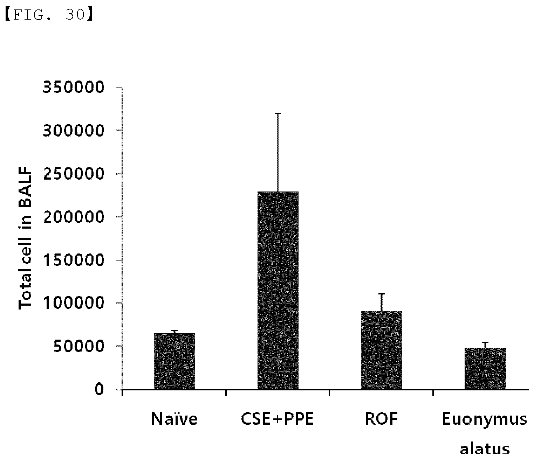

After injecting 1 mL of PBS through the airway of the mouse, it was gently massaged to recover 700 µL of bronchoalveolar lavage fluid (BALF). 10 uL of the recovered BALF solution was mixed with an Accustain T solution in the same amount and total cells were automatically counted with a cell counter (ADAM-MC NANOENTEK. INC, Korea) after injecting 12 uL into the Accuchip channel. The results according to the administration of each extract were shown in FIGS. 22 to 30.

### 4-3: Analysis of immune cells in BALF through Diff-Quick staining

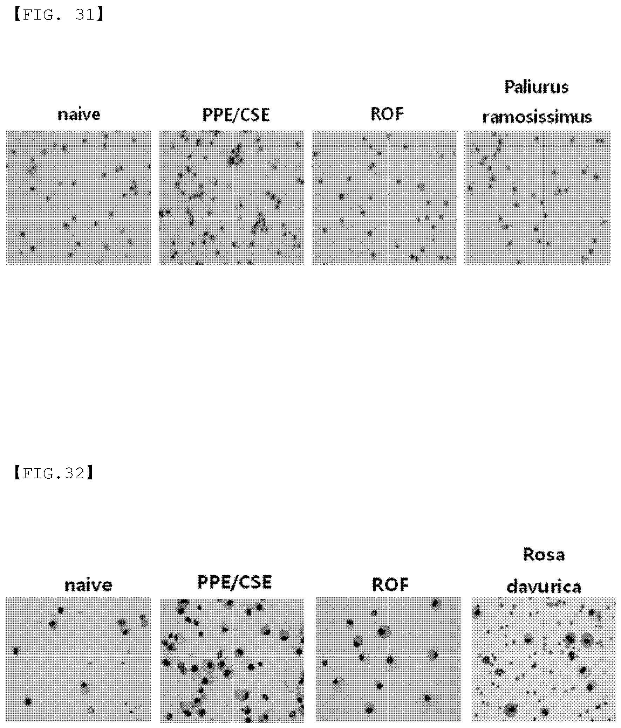

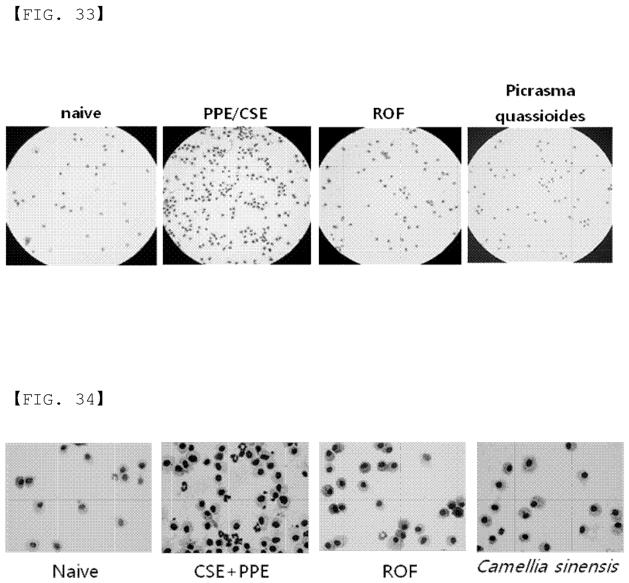

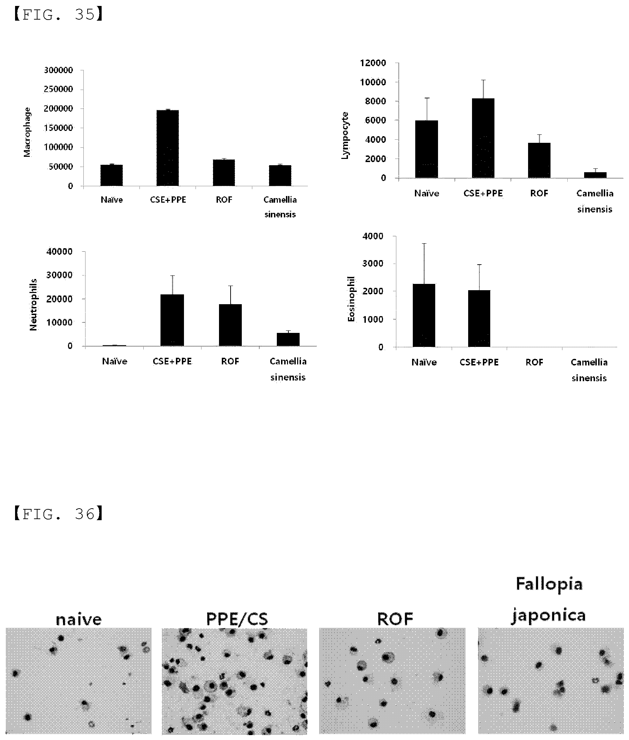

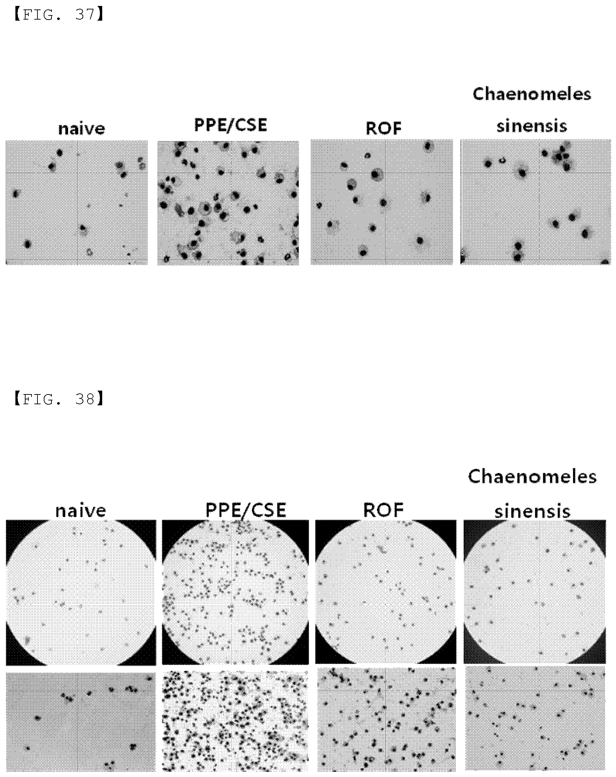

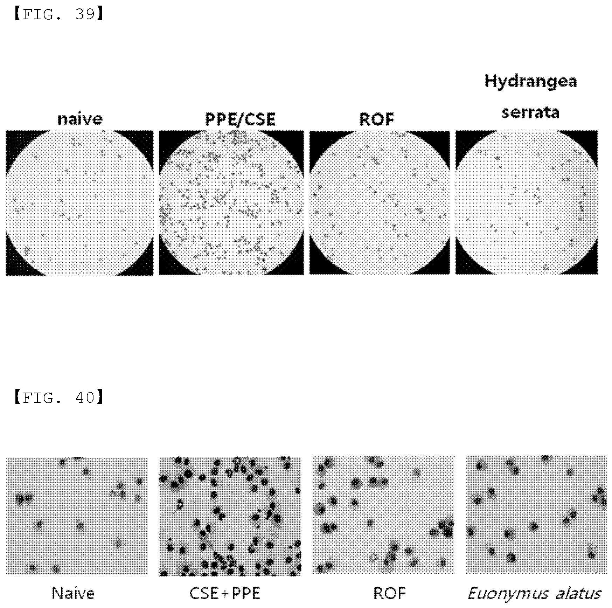

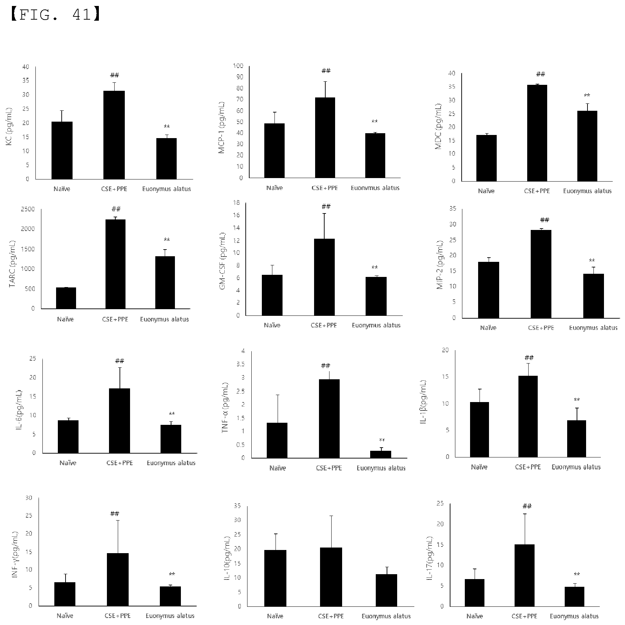

The recovered BALF was separated into a supernatant and a cell pellet through centrifugation under conditions of 300×g and 5 minutes. The cell pellet was resuspended by adding 700 µL of PBS, and 150 µL of this BALF suspension was centrifuged with a Cytospin device (Centrifuge 5403, Eppendorf, Hamburg, Germany) under conditions of 1000 rpm, 10 min, 4°C, and the BALF cells were attached to the slide. Thereafter, the cells were stained according to the protocol of the manufacturer (1-5-1 Wakinohamakaigandori, chuo-ku, Kobe, Japan) using a Diff-Quick staining reagent, and the number of Macrophages, Lympocytes, Neutrophils, and Eosinophils were counted through microscopic observation. The results according to the administration of each extract were shown in FIGS. 31 to 41.

### 4-4: Histological examination

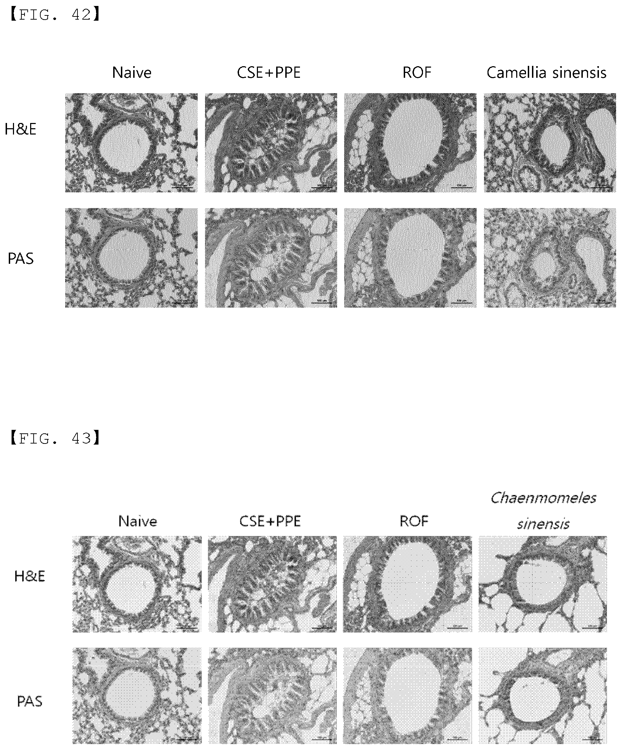

Lung tissue was extracted from the dissected mouse, the mesenchyme of the lung was removed, and then fixed in 10% neutral buffered formalin for 3 days. The fixed tissue was dehydrated with ethyl alcohol and xylene, embedded with paraffin, trimmed, and cut into 5 um thickness. After attaching the prepared section to the slide, it was stained with H&E and observed under a microscope in order to confirm the general shape. The results according to the administration of each extract were shown in FIGS. 42 to 44.

### 4-5: Analysis of Cytokines in BALF

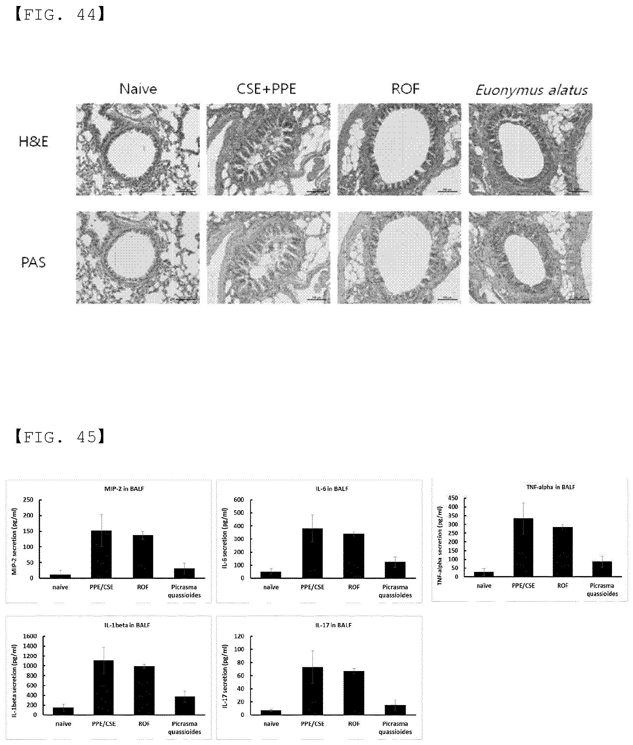

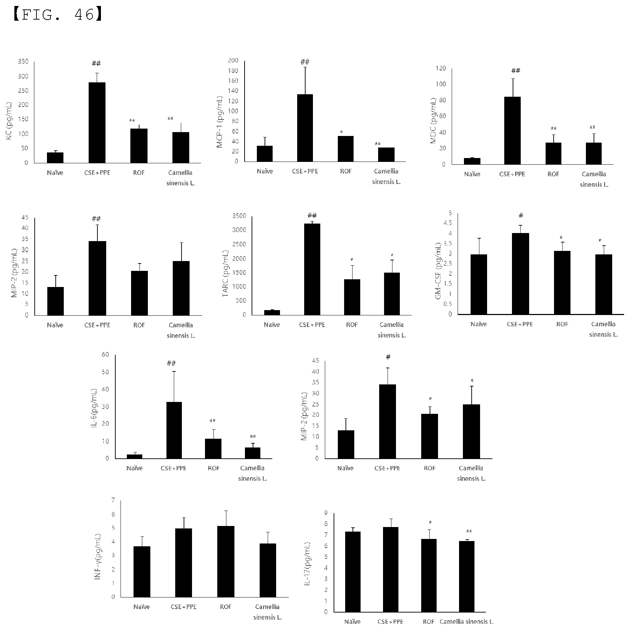

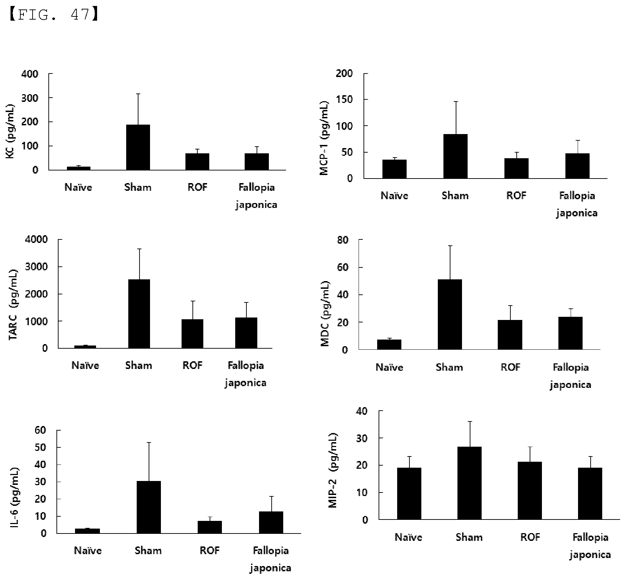

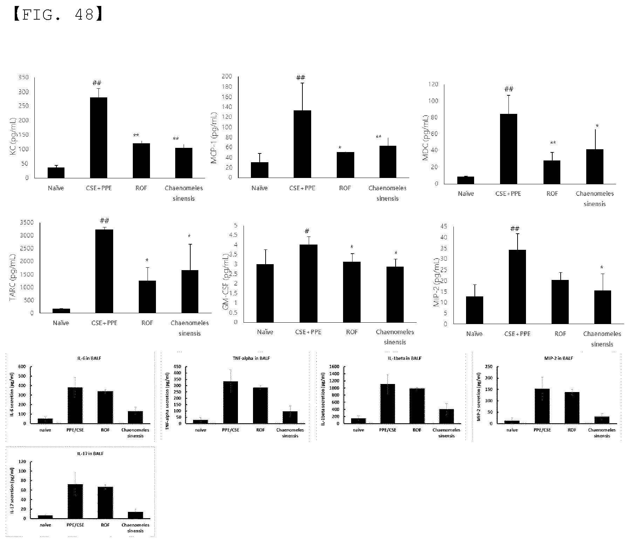

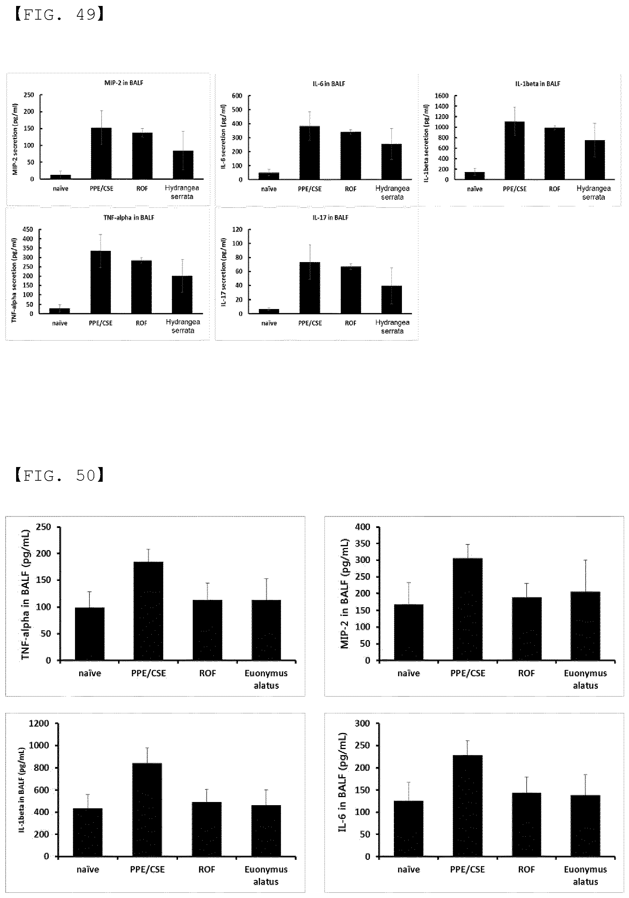

After injecting 1 mL of PBS through the airway of the mouse, it was gently massaged to recover 700 µL of bronchoalveolar lavage fluid (BALF). The recovered BALF was centrifuged at 300×g for 5 minutes to separate the supernatant and then analyzed for cytokines and chemokines (TARC, KC, MCP-1, MDC, GM-CSF, MIP-2, TNF-α, IL-6, IL-1β, IL-17, IL-10, IFN-γ). After taking 30 uL of the supernatant for analysis of cytokines and chemokines, the experiment was performed according to the protocol of the manufacturer of the Q-plex ELISA array kit (Quansis biosciences, Inc.). The results according to the administration of each extract were shown in FIGS. 45 to 50.

### 4-6: Analysis of Cytokines in Lung Tissue

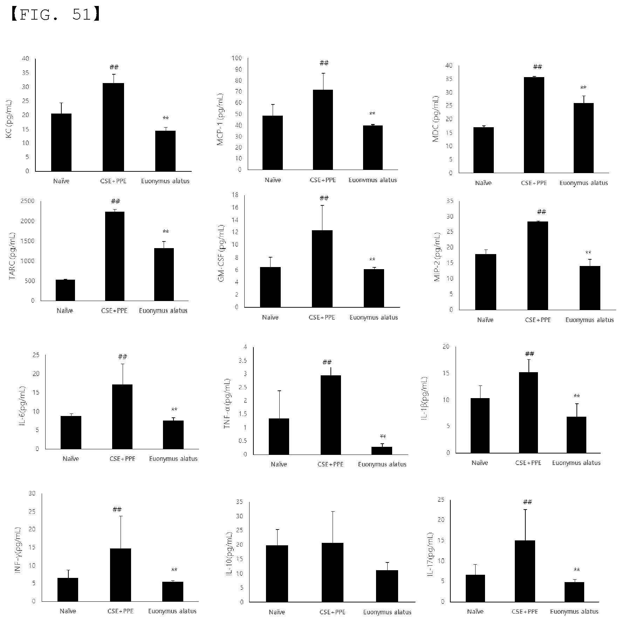

PBS corresponding to 10 times the weight of the lung tissue was added, pulverized with a homogenizer, and centrifuged at 12,000 rpm for 20 min. The recovered supernatant was used for analysis of cytokines and chemokines (TARC, KC, MCP-1, MDC, GM-CSF, MIP-2, TNF-α, IL-6, IL-1β, IL-17, IL-10, IFN-γ). After taking 30 uL of the supernatant for analysis of cytokines and chemokines, the experiment was performed according to the protocol of the manufacturer of the Q-plex ELISA array kit (Quansis biosciences, Inc.) (FIG. 51).

From the above description, those skilled in the art to which the present disclosure pertains will understand that the present disclosure can be implemented in other specific forms without changing the technical spirit or essential features thereof. In this connection, it should be understood that the Examples described above are illustrative in all respects but not limiting. The scope of the present disclosure should be construed as including all changes or modifications derived from the meaning and scope of the claims to be described later rather than the above detailed description, and equivalent concepts thereof.

## Claims

1. A food composition for reinforcing respiratory function and alleviating respiratory disease, comprising one or more of effective ingredients selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata, and Euonymus alatus.

2. The composition of claim 1, wherein the extract is extract obtained by using water, ethanol or a mixed solvent thereof.

3. The composition of claim 1, wherein the respiratory disease is a lung disease accompanied by cough, sputum, respiratory distress, airway hypersensitivity, airway obstruction, mucus hypersecretion, decreased expiratory flow, and/or impaired gas exchange.

4. The composition of claim 1, wherein the respiratory disease is asthma, COPD, diffuse interstitial lung disease, acute respiratory distress syndrome (ARDS), or acute lung injury.

5. The composition of claim 4, wherein the respiratory disease is asthma or COPD.

6. A healthy functional food, comprising the food composition of any one of claims 1 to 5.

7. A pharmaceutical composition for preventing or treating respiratory disease, comprising one or more of effective ingredients selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata, and Euonymus alatus.

8. The composition of claim 7, wherein the extracts are extract obtained by using water, ethanol or a mixed solvent thereof.

9. The composition of claim 7, wherein the respiratory disease is asthma, COPD, diffuse interstitial lung disease, acute respiratory distress syndrome (ARDS), or acute lung injury.

10. The composition of claim 9, wherein the respiratory disease is asthma or COPD.

11. An anti-allergic food composition, comprising one or more of effective ingredients selected from the group consisting of extracts of Paliurus ramosissimus, Rosa davurica, Picrasma quassioides, Camellia sinensis, Chrysosplenium grayanum, Reynoutria elliptica, Chaenomeles sinensis, Hydrangea serrata, and Euonymus alatus.

12. A healthy functional food, comprising the food composition of claim 11.
